(19) Europäisches Patentamt
European Patent Office
Office européen des brevets

(11) **EP 2 874 547 B1**

(12) **EUROPÄISCHE PATENTSCHRIFT**

(45) Veröffentlichungstag und Bekanntmachung des Hinweises auf die Patenterteilung:
**27.04.2016 Patentblatt 2016/17**

(21) Anmeldenummer: **13737211.6**

(22) Anmeldetag: **10.07.2013**

(51) Int Cl.:
***A61B 17/16*** *(2006.01)*

(86) Internationale Anmeldenummer:
**PCT/EP2013/064615**

(87) Internationale Veröffentlichungsnummer:
**WO 2014/012833 (23.01.2014 Gazette 2014/04)**

(54) **ANTRIEBSSTEUEREINRICHTUNG UND -VERFAHREN FÜR EIN CHIRURGISCHES MOTORENSYSTEM**

DRIVE CONTROL DEVICE AND METHOD FOR A SURGICAL MOTOR SYSTEM

DISPOSITIF ET PROCÉDÉ DE COMMANDE D'ENTRAÎNEMENT D'UN SYSTÈME CHIRURGICAL À MOTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.07.2012 DE 102012106589**

(43) Veröffentlichungstag der Anmeldung:
**27.05.2015 Patentblatt 2015/22**

(73) Patentinhaber: **Aesculap AG**
**78532 Tuttlingen (DE)**

(72) Erfinder:
• **SCHNEIDER, Jürgen**
**78532 Tuttlingen (DE)**
• **KONRATH, Harald**
**72108 Rottenburg-Hailfingen (DE)**

(74) Vertreter: **Winter, Brandl, Fürniss, Hübner, Röss, Kaiser, Polte - Partnerschaft mbB Patent- und Rechtsanwaltskanzlei Bavariaring 10 80336 München (DE)**

(56) Entgegenhaltungen:
**EP-A1- 2 324 779**

**Beschreibung**

Die vorliegende Erfindung betrifft eine Antriebssteuereinrichtung für ein chirurgisches Motorensystem gemäß dem Oberbegriff des Anspruchs 1, die eine Motorsteuerung zum Steuern und/oder Regeln eines ein chirurgisches Werkzeug antreibenden Elektromotors aufweist. Ein weiterer Aspekt der Erfindung betrifft das Motorensystem selbst.

Ferner betrifft die vorliegende Erfindung ein Verfahren zum Ansteuern eines chirurgisches Motorensystems gemäß dem Oberbegriff des Anspruchs 11, bei dem das chirurgische Werkzeug von einem Elektromotor angetrieben wird, der mittels einer Motorsteuerung gesteuert und/oder geregelt wird.

Chirurgische Motorensysteme der gattungsgemäßen Art sind in zahlreichen Varianten bekannt, insbesondere mit Werkzeugen in Form von Bohr- und Fräsmaschinen oder Sägen. Sie werden betrieben, indem mit der Motorsteuerung Steuersignale für den Elektromotor erzeugt werden, um ihn mit einer bestimmten, von der Antriebssteuereinrichtung vorgebbaren Drehzahl zu betreiben. Je nach Art des Elektromotors können Drehzahlen bis zu 80.000 Umdrehungen pro Minute erreicht werden. Besonders kostengünstig und wenig wartungsintensiv wird ein solches Motorensystem, wenn der Elektromotor ein bürstenloser Gleichstrommotor ist, der neben dem Rotor mindestens zwei Motorwicklungen aufweist.

Antriebssteuereinrichtungen für chirurgische Motorensysteme der gattungsgemäßen Art sind beispielsweise aus der WO 2006/012991 A1 oder der DE 10 2009 018 143 A1 bekannt. Bei diesen bekannten Antriebssteuereinrichtungen wird die Drehzahl des betreffenden chirurgischen Werkzeugs geregelt, wobei z.B. eine Puls-Weiten-Modulation (PWM) oder eine Space-Vektor-Puls-Weiten-Modulation (SVPWM) zum Einsatz kommt. Das SVPWM-Verfahren hat gegenüber der herkömmlichen Puls-Weiten-Modulation den Vorteil, dass alle Motorwicklungen gleichzeitig bestrombar sind, so dass auch bei besonders niedrigen Drehzahlen ein sanfter, ruckelfreier Betrieb des Elektromotors möglich ist.

Aus EP 2 324 779 A1 ist ein chirurgisches Motorensystem für unterschiedliche medizinische Applikationen bekannt, die eine Antriebssteuerung mit einer elektronischen Schaltung aufweist, welche zum Steuern bzw. Regeln der Drehzahl des Motorensystems dient. Die Drehzahlsteuerung bzw. -regelung ändert sich in Abhängigkeit des Werkzeughalters, welcher an dem chirurgischen Motorensystem angebracht wird

Diese bekannten Regelungsverfahren zeichnen sich neben ihrem sanften Betrieb vor allem aber auch dadurch aus, dass die jeweilige Drehzahl sehr schnell nachgeregelt werden kann. Es hat sich aber gezeigt, dass z.B. bei extremen Belastungsänderungen ein sehr schnelles Nachregeln der Drehzahl nicht nur Vorteile nach sich zieht, wie man aus folgender Gegenüberstellung erkennt:

Wenn z.B. ein ungeregelter, mit Druckluft betriebener Motor beim Antreiben eines Fräsers einen kurzzeitigen Drehzahleinbruch als Folge eines eventuellen Verhakens des Fräsers erfährt, so hat dies zur Folge, dass nach dem Lösen des Fräsers die Motordrehzahl wieder steigt. Es entsteht dabei keine zusätzliche Erwärmung.

Wenn ein solcher Fräser demgegenüber durch einen geregelten Elektromotor angetrieben wird, regelt die Motorsteuerung in einer solchen Situation sehr schnell nach, um dem Drehzahleinbruch entgegen zu wirken. Dies führt insbesondere dann zu Problemen, wenn sich das Werkzeug mehrmals nacheinander verhakt und die Drehzahl immer wieder nachgeregelt werden muss. Wenn man den Stromverlauf in einer solchen Situation näher untersucht, kann man zusätzliche Bestromungsimpulse messen ($I^2$ x R). Folglich treten bei diesen sehr schnellen, dynamischen Regelvorgängen zusätzliche Verluste auf. Durch das ständige Nachregeln und die damit verbundenen Stromspitzen produziert der Elektromotor mehr Wärme und kann so überhitzen.

Es gibt viele Anwendungen (z. B. bei einer Kraniotomie), bei denen ein solch schnelles Nachregeln erwünscht bzw. sogar erforderlich ist, wobei der geschilderte Nachteil der zusätzlichen Verluste aufgrund der vergleichsweise kurzen Aktivierungszeiten bei einer Kraniotomie ohne weiteres in Kauf genommen werden kann. Es gibt allerdings auch Anwendungen, wie z.B. das zeitintensive Abfräsen eines Knochensegmentes, bei dem weniger das schnelle Nachregeln, als vielmehr die geringst mögliche Verlustwärme im Fokus steht.

Wenn die vorstehend erwähnten, z.B. aus der WO 2006/012991 A1 oder der DE 10 2009 018 143 A1 bekannten Antriebssteuereinrichtungen beim Abfräsen eines Knochensegmentes verwendet werden, besteht daher die Gefahr, dass der Elektromotor überhitzt oder ggf. sogar beschädigt wird. Demnach ist der Einsatzbereich der bislang bekannten Antriebssteuereinrichtungen für chirurgische Instrumente oder Geräte aus Sicherheitsgründen auf bestimmte Einsatzzwecke beschränkt. Daher werden bislang je nach Anwendungsbereich unterschiedliche chirurgische Motorensysteme bzw. solche mit jeweils geeigneten Antriebsmotoren verwendet. Dies erschwert die Handhabung und erhöht zudem die Kosten. Wünschenswert wäre jedoch, wenn ein Motorensystem für verschiedenste medizinischen Applikationen eingesetzt werden könnte.

Der Erfindung liegt die Aufgabe zugrunde, eine Antriebssteuereinrichtung gemäß Oberbegriff des Anspruchs 1 so weiterzubilden, dass der Einsatzbereich des zugeordneten chirurgischen Motorensystems vergrößert werden kann. Ebenfalls soll ein entsprechendes Steuerungsverfahren sowie ein chirurgisches Motorensystem angegeben werden.

Diese Aufgabe wird hinsichtlich der Antriebssteuereinrichtung mit den Merkmalen des Anspruchs 1, hinsichtlich des chirurgischen Motorensystems mit den Merkmalen des Anspruchs 9 und hinsichtlich des Verfahrens mit den Verfahrensschritten des Anspruchs 11 gelöst. Vorteilhafte Weiterbildungen sind jeweils Gegenstand von Unteransprüchen.

Die Erfindung schlägt demnach vor, eine der Motorsteuerung vorgeschaltete Parameter-Vorgabeeinrichtung vorzusehen, mit der die Steuer- bzw. Regelungsparameter der Motorsteuerung so vorgegeben werden können, dass das Steuerungs- bzw. Regelungsprofil des Elektromotors dem jeweiligen Zustand des Elektromotors anpassbar ist. Die Erfindung basiert somit auf dem Gedanken, anstatt für unterschiedliche Anwendungen unterschiedliche Motorensysteme zu verwenden, den Steuerungsmodus bzw. das Steuerungs- bzw. Regelungsprofil des Elektromotors so zu variieren, dass es der jeweiligen Anwendung optimal angepasst ist, um so einen größeren Einsatzbereich des verwendeten Motorensystems zu ermöglichen. Die Grundlage für die Auswahl der Steuer- bzw. Regelungsparameter bildet ein erfasster Zustand bzw. Zustandsverlauf des Elektromotors, anhand dessen die Parameter-Vorgabeeinrichtung die momentane medizinische Applikation ermitteln kann. Dadurch wird bei geeigneter Parameterwahl erreicht, dass das sich ergebende Steuerungs- bzw. Regelungsprofil eine Überlastung, wie z.B. eine unzulässige Erwärmung des Elektromotors, sicher verhindert. Die erfindungsgemäße Ansteuerung kann daher für die verschiedensten chirurgischen Zwecke eingesetzt werden, ohne dass unterschiedliche Motoren benötigt werden. Die unterschiedlichen Steuerungsmodi bzw. Ansteuerungsprofile können rein softwaretechnisch realisiert werden, während die Hardware, d.h. die Steuerelektronik und der Motor, in unveränderter Weise bestehen bleiben können. Die Handhabbarkeit wird dadurch deutlich verbessert und die Kosten verringern sich entsprechend.

Durch Änderung der Steuer- bzw. Regelungsparameter kann das Steuerungs- bzw. Regelungsprofil des Elektromotors so geändert werden, dass die sich ergebende Drehmoment/Drehzahl-Kennlinie dem jeweiligen Belastungszustand optimal angepasst ist.

Ein Steuerungsmodus im Sinne der Erfindung beeinflusst oder ändert die Sollwerte bzw. Führungsgröße und/oder die Steuerungs- bzw. Regelcharakteristik selbst. So kann z.B. in einem Anwendungsfall, bei dem kein sehr schnelles Nachregeln notwendig ist wie z.B. beim Abfräsen eines Knochensegments in der Regelung der P-Anteil reduziert und der I-Anteil erhöht werden, um so hohe Stromspitzen zu vermeiden. Alternativ oder zusätzlich kann das Niveau des Maximalstroms insgesamt abgesenkt werden. Ferner kann durch die Parameter-Vorgabeeinrichtung der Sollwert bzw. die Solldrehzahl entsprechend abgesenkt werden, insbesondere nach Überschreitung oder bei zu erwartender Überschreibung eines Grenzstroms.

Vorzugsweise kann der Elektromotor bzw. das Werkzeug zunächst gemäß einem Standardsteuerungsmodus oder einem manuell einstellbaren Steuerungsmodus betrieben werden und anschließend in Abhängigkeit des erfassten Zustandes bzw. der erkannten momentanen medizinischen Applikation auf einen hierfür geeigneteren Steuerungsmodus geändert bzw. umgeschaltet werden.

Je nach Anwendungsfall kann auch zwischen einem Betrieb mit Drehzahlregelung und einem Betrieb ohne Drehzahlregelung (reine Steuerung) umgeschaltet werden, können für die Drehzahlregelung unterschiedliche Regelparameter verwendet werden oder kann bei unveränderten Regelparametern die Sollwertgröße situationsabhängig angepasst werden. Eine weitere Alternative stellt die Änderung des Motorinnenwiderstandes dar, bei der proportional zum gemessenen Strom die Ausgangsspannung für den Motor entsprechend abgesenkt oder erhöht wird.

Die unterschiedlichen Steuerungs- bzw. Regelungsprofile sind in Form von Steuerungsmodi hinterlegt, aus welchen ein geeigneter situationsabhängig ausgewählt werden kann. Erfindungsgemäß erfolgt die Auswahl und Umschaltung automatisch.

Erfindungsgemäß wird hierzu der jeweilige Zustand des chirurgischen Motorensystems dynamisch erfasst, um das Steuerungs- bzw. Regelungsprofil entsprechend den jeweiligen Erfordernissen quasi kontinuierlich ändern zu können.

Gemäß einer Ausführungsform können eine Vielzahl unterschiedlicher Steuerungsmodi, d.h. Steuer- und/oder Regelungsparameter bzw. Motorkennlinien hinterlegt sein, die jeweils entsprechenden medizinischen Applikationen zugeordnet sind. Die Parameter-Vorgabeeinrichtung kann dann in Abhängigkeit des erfassten Zustandes bzw. Zustandsverlaufs des Elektromotors aus diesen den passenden Steuerungsmodus auswählen.

Alternativ oder zusätzlich kann die Auswahl des Steuerungsmodus in Abhängigkeit des verwendeten chirurgischen Werkzeugs oder Werkzeugadapters erfolgen. Durch die vorzugsweise automatische Erkennung kann ein oder mehrere für das Werkzeug passende Steuerungsmodus ausgewählt werden. Beispielsweise werden unterschiedliche Werkzeug mit unterschiedlichen Drehzahlen gefahren, z.B. mit max. 20.000 U/min oder mit bis zu max. 80.000 U/min. Durch die Erkennung des Werkzeugs wird sichergestellt, dass die maximale Motordrehzahl und/oder das maximale Drehmoment entsprechend begrenzt werden.

Jedem Steuerungsmodus kann eine vorbestimmte Drehmoment/Drehzahl-Kennlinie des Elektromotors zugeordnet sein.

Anstelle von oder zusätzlich zu fest hinterlegten Steuerungsmodi kann ein passender Steuerungsmodus auf der Grundlage eines Lernalgorithmus generiert werden.

Der Steuerungsmodus kann in einer Ausführungsform auch in Abhängigkeit von einer erfassten Regelungshäufigkeit pro Zeiteinheit auswählt werden, die auf eine Applikation mit vielen Belastungsschwankungen schließen lässt.

Es kann ferner vorteilhaft sein, den jeweiligen Zustand des chirurgischen Motorensystems durch, vorzugsweise kontinuierliche, Erfassung des jeweiligen Momentanwerts von Motor-Strom und Motor-Spannung des Elektromotors zu bestimmen. Wenn als Motorsteuerung ohnehin ein Regler vorgesehen ist, hat dies den Vorteil, dass die für diese Regelung erforderlichen Sensoren bereits vorhanden sind, so dass keine zusätzlichen Kosten entstehen.

Der jeweilige Zustand des chirurgischen Motorensystems kann ferner oder alternativ durch, vorzugsweise kontinuierliche, Erfassung einer Temperatur des Elektromotors (M) bestimmt werden, wobei die Temperatur bei Fehlen eines entsprechenden Sensors durch Integration der jeweiligen Momentanwerte von Motor-Strom und Motor-Spannung auch indirekt erfasst werden kann.

Vorteilhaft ist es auch, wenn zumindest ein Steuerungsmodus eine Drehzahlregelung des Elektromotors vorsieht und zumindest ein anderer Steuerungsmodus keine Drehzahlregelung des Elektromotors vorsieht. So kann softwaretechnisch sowohl ein geregelter als auch ein ungeregelter Betrieb, insbesondere der ungeregelte Betrieb eines Druckluftmotors simuliert werden.

Die Motorsteuerung kann beim Steuer- und/oder Regelungsverfahren eine Puls-Weiten-Modulation oder eine Space-Vektor-Puls-Weiten-Modulation (SVPWM), bei der alle Motorwicklungen gleichzeitig bestromt werden, durchführen.

Um den Zustand des Elektromotors feststellen zu können, kann eine Rotorpositionsbestimmungseinrichtung zum Bestimmen einer Rotorposition des Elektromotors vorgesehen sein, wobei zur Bestimmung der Position des Rotors des Elektromotors mindestens eine der Motorwicklungen für ein vorbestimmtes Zeitintervall von einer Energieversorgung abgetrennt wird. Bei einer Drehzahl von 80.000 U/min und drei jeweils nacheinander abgeschalteten Motorwicklungen kann die Position des Rotors und die elektromotorische Kraft 4.000 mal pro Sekunde gemessen werden. Dadurch lässt sich der Zustand des Elektromotors sehr schnell erfassen und ein entsprechender Steuerungsmodus ausgewählt werden.

Ein erfindungsgemäßes chirurgisches Motorensystem weist einen Elektromotor zum Antrieb eines chirurgischen Werkzeugs, das mit diesem unmittelbar oder mittelbar, insbesondere unter Zwischenschaltung eines Werkzeugadapters oder Handstücks, lösbar koppelbar ist, sowie eine zuvor beschriebene Antriebssteuereinrichtung auf.

Zusätzlich kann das chirurgische Motorensystem eine Drehzahlerfassungseinrichtung zum Erfassen der Ist-Drehzahl des Elektromotors, insbesondere Hall-Sensoren, und/oder eine Handstück- oder Werkzeugerkennungseinrichtung aufweisen. Dadurch kann der Zustand des Elektromotors bzw. das verwendete Werkzeug- oder Werkzeugsystem automatisch erkannt werden und ein entsprechender Steuerungsmodus ausgewählt werden.

Das erfindungsgemäße Verfahren zum Steuern und/oder Regeln eines Elektromotors (M) eines chirurgisches Motorensystems, der ein chirurgisches Werkzeug antreibt, ist durch die folgenden Schritte: Erfassen eines Zustands und/oder Zustandsverlaufs des Elektromotors des chirurgischen Motorensystems; Auswählen eines Steuerungsmodus auf der Grundlage des erfassten Zustandes oder Zustandsverlaufs; und Einstellen der Steuer- bzw. Regelungsparameter, so dass das Steuerungs- bzw. Regelungsprofil des Elektromotors dem ausgewählten Steuerungsmodus entspricht. Bezüglich noch weiterer Vorteile und Weiterbildungen der Erfindung wird auf die Unteransprüche verwiesen.

Die Erfindung wird nachstehend anhand der Beschreibung eines Ausführungsbeispiels unter Bezugnahme auf die Zeichnung näher erläutert. Es zeigen:

Fig. 1    ein Blockschaltbild eines chirurgischen Motorensystems mit einer erfindungsgemäßen Antriebssteuereinrichtung;

Fig. 2    ein Blockschaltbild einer erfindungsgemäßen Parameter-Vorgabeeinrichtung PV; und

Fig. 3    mehrere Kennlinien, die als Steuerungs- bzw. Regelungsprofil des chirurgischen Motorensystems realisiert werden.

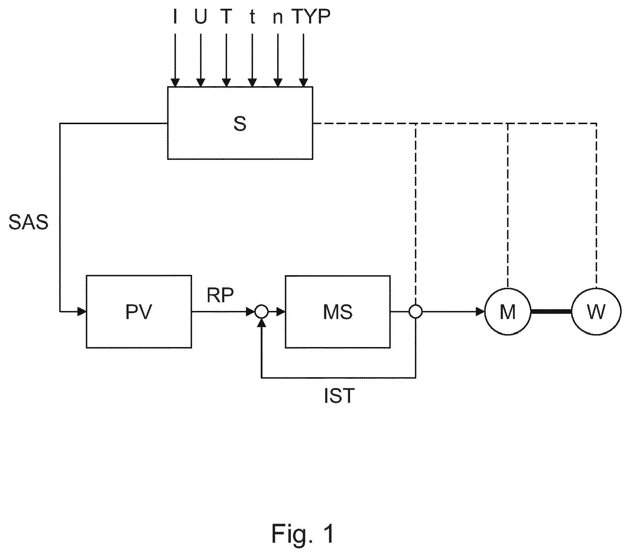

Gemäß Fig.1 umfasst das erfindungsgemäße chirurgische Motorensystem einen Elektromotor M, der mechanisch über eine Werkzeugkupplung mit einem Werkzeug W gekoppelt ist und dieses antreibt;bei dem Werkzeug W kann es sich um einen Bohrer, Fräser, eine Säge, ein Kraniotomie- oder Trepanationswerkzeug, einem Shaver oder ein anderes chirurgisches Werkzeug handeln. Die grundsätzliche Ansteuerung des Werkzeugs erfolgt über eine (nicht gezeigte) Hand- oder Fußsteuerung. Der Elektromotor M wird dabei von einer Motorsteuerung MS angesteuert. Diese erzeugt für jede Phase bzw. Wicklung des Elektromotors M entweder ein Puls-Weiten-Modulations-Signal (PWM) oder ein Space-Vektor-Puls-Weiten-Modulations-Signal (SVPWM), wobei im Falle des letzteren alle Motorwicklungen gleichzeitig bestromt werden.

Wenn sich die Motorsteuerung MS im Modus einer Drehzahlregelung befindet, wird ihrem Eingang ein Istwert-Signal IST zugeführt, das den für die Regelung erforderlichen Momentanwert der Drehzahl angibt. Zur Erfassung der Drehzahl sind entsprechende (nicht gezeigte) Sensoren, insbesondere in Form von Hall-Sensoren vorgesehen. Wenn für die von der Motorsteuerung MS durchgeführte Regelung auch andere Rückkopplungsgrößen benötigt werden, wie insbesondere den zugeführten Strom oder die anliegende Spannung, enthält das Istwert-Signal IST entsprechende Informationen.

Der Motorsteuerung MS ist gemäß Fig. 1 eine Parameter-Vorgabeeinrichtung PV vorgeschaltet; diese erzeugt Steuer- bzw. Regelungsparameter RP, die als Führungsgröße neben dem Istwert-Signal IST am Eingang der Motorsteuerung MS anliegen. Durch die Art der jeweils anliegenden Steuer- bzw. Regelungsparameter RP wird das von der Motorsteuerung MS bereitgestellte Steuerungs- bzw. Regelungsprofil eingestellt bzw. vorgegeben. Zunächst erfolgt die Steuerung bzw. Regelung des Werkzeugs W gemäß einem Standardsteuerungsmodus oder von einem Benutzer manuell eingegebenen Steuerungsmodus.

Gemäß Fig. 1 ist weiterhin eine Sensorik S vorgesehen, an deren Eingängen die Signale mehrerer Sensoren anliegen, nämlich ein den momentanen Motorstrom repräsentierendes Signal I, ein die Motorspannung darstellendes Signal U sowie ein Drehzahl-Signal n. Letzteres repräsentiert die momentane Drehzahl des Elektromotors M und wird, wie bereits erwähnt, beispielsweise von einem Hall-Sensor erzeugt. Ferner kann noch ein von einem Temperaturfühler erzeugtes Signal T, das die momentane Temperatur des Elektromotors M angibt. Da im chirurgischen Bereich die jeweiligen Maschinen bzw. Werkzeuge sehr klein sind, ist das Plazieren eines solchen Temperaturfühlers sowie die Weiterleitung seines Ausgangssignals T oft mit großen Schwierigkeiten verbunden. Das Temperatursignal T wird in einer Ausführungsform der Erfindung daher durch Integration der erfassten Momentanwerte von Motor-Strom und Motor-Spannung des Elektromotors M bestimmt. Schließlich kann noch die Dauer der momentanen medizinischen bzw. chirurgischen Behandlung, d.h. die momentane Dauer des momentanen Werkzeugeinsatzes, gemessen werden und als Zeitsignal t ausgegeben werden.

Desweiteren kann das Motorensystem noch eine Sensorik aufweisen, welche die Art des momentan verwendeten Werkzeugs automatisch erkennt und ein entsprechendes Typensignal TYP ausgibt. Die Sensorik erzeugt gemäß Fig. 1 ein Sensor-Ausgangssignal SAS, das der Parameter-Vorgabeeinrichtung PV zugeführt wird.

In der Parameter-Vorgabeeinrichtung PV oder einen anderen Speichereinrichtung sind anwendungstypische Zustandsgrößen und -verläufe der Eingangssignale U, I, n, T; t, TYP hinterlegt. Durch verschiedene Versuchs- und Messreihen im Vorfeld oder durch einen Lernalgorithmus kann ermittelt werden, welche Zustandsgrößen und -verläufe für die jeweiligen Werkzeugeinsätze typisch sind. Diese Zuordnung von Zustandsgrößen und medizinischer Applikation ermöglicht es der Parameter-Vorgabeeinrichtung PV, anhand der aktuellen Eingangsgrößen bzw. Messwerte die momentane Applikation zu ermitteln.

Wie eingangs beschrieben bedarf jede Applikation bzw. Werkzeugeinsatz unterschiedliche Antriebe. Bei Fräsanwendungen sind beispielsweise kleinere Motorströme im Bereich von max. 1 A und Momente von max. 1,5 Ncm zu erwarten, während bei einer Kraniotomie eher Ströme von bis zu ca. 3,5 A und Momente von bis zu 3,5 Ncm auftreten können.

Neben der automatischen Erkennung der momentanen Applikation wählt die Parameter-Vorgabeeinrichtung PV in einem zweiten Schritt den hierfür passenden Steuerungsmodus aus. Diese Korrelation von Applikation und Steuerungsmodus ist ebenfalls in der Parameter-Vorgabeeinrichtung PV oder einen anderen Speichereinrichtung hinterlegt.

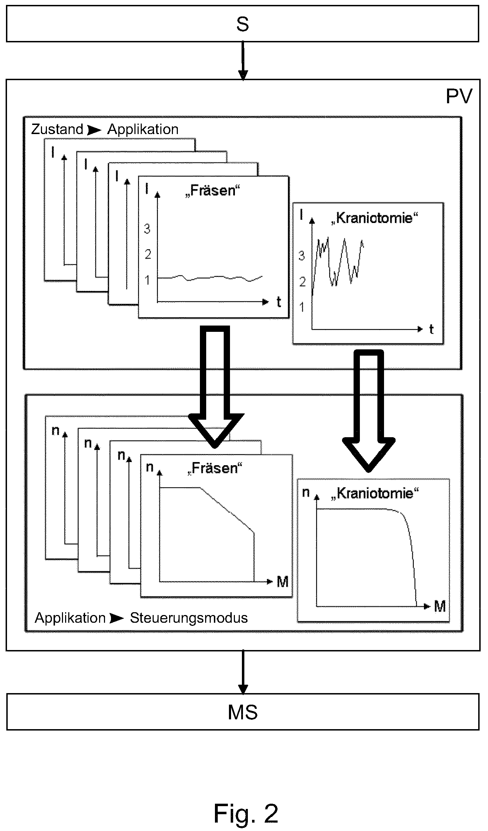

Diese zwei von der Parameter-Vorgabeeinrichtung PV durchgeführten Schritte sind schematisch in der Fig. 2 am Beispiel von den "Fräsen" und "Kraniotomie" auf der Grundlage eines zeitlichen Verlaufs des Stroms I gezeigt. Um die Zuordnungsverlässlichkeit zu erhöhen, können mehrere oder alle gemessenen bzw. ermittelten Werte mit entsprechenden hinterlegten Verläufen verglichen und ausgewertet werden.

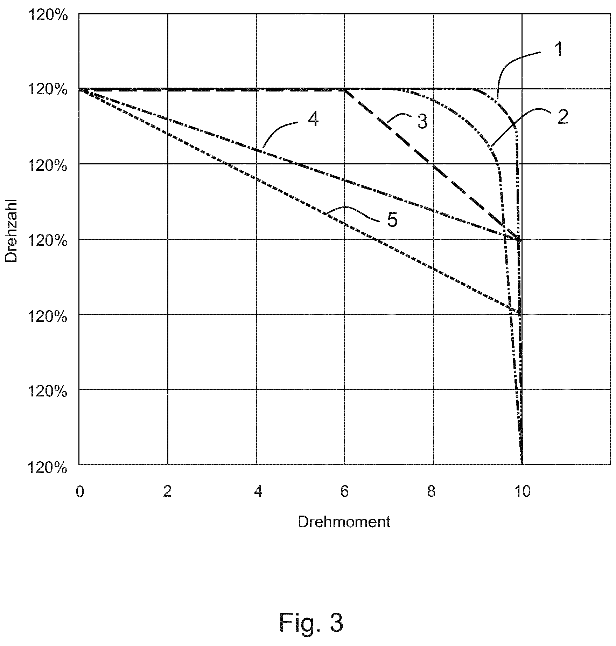

Nach der Auswahl eines applikationsorientierten Steuerungsmodus gibt die Parameter-Vorgabeeinrichtung PV an die Motorsteuerung MS entsprechende Steuerungs- und/oder Regelungsparameter aus und verändert so das Steuerungs- bzw. Regelungsverhalten der Motorsteuerung MS. Die Parameter-Vorgabeeinrichtung PV schaltet somit von dem zunächst standardmäßig oder manuell eingestellten Steuerungsmodus automatisch in einen geeigneteren Steuerungsmodus um. Das heißt, die Parameter-Vorgabeeinrichtung PV ist in der Lage, der Motorsteuerung MS ein solches Steuerungs- bzw. Regelungsprofil einzuprägen, das dem durch die Signale U, I, n, T, t und/oder TYP repräsentierten momentanen Belastungszustand des Elektromotors M am besten gerecht wird. Wenn beispielsweise anhand der Messwerte bzw. deren zeitlichen Verlauf erkannt wird, dass mit dem chirurgischen Werkzeug W gerade eine Kraniotomie durchgeführt wird, wird von der Parameter-Vorgabeeinrichtung PV an der Motorsteuerung MS ein Regelungsprofil eingestellt, das z.B. die Motorkennlinie 1 in der Fig. 3 zur Folge hat, d.h. der Elektromotor M wird mit voller Kraft in seiner Drehzahl konstant gehalten bzw. schnellstmöglich nachgeregelt. Wenn demgegenüber erkannt wird, dass es sich bei der mit dem Werkzeug W durchgeführten Tätigkeit eher um das zeitintensive Abfräsen eines Knochensegmentes handeln könnte, wird vorzugsweise eine der Kennlinien 3 bis 5 in der Fig. 3 eingestellt, bei denen die Erwärmung des Elektromotors M weitaus geringer ist.

In der Fig. 3 sind beispielhaft einige Motorkennlinien in Form des Drehmoment/Drehzahl-Verlaufs des Elektromotors M gezeigt, die die Motorsteuerung MS dem Elektromotor M in Abhängigkeit vom jeweiligen Steuer- bzw. Regelungsparameter RP der Parameter-Vorgabeeinrichtung PV einprägt. Die mit 1 bezeichnete Kennlinie ist z.B. eine Drehzahlregelungs-Kennlinie, bei der die Drehzahl bis auf einen sehr kleinen Bereich ab 9,5 Ncm konstant gehalten werden kann. Die mit 2 und 3 bezeichneten Kennlinien sind Drehzahlregelungs-Kennlinien, bei der die Drehzahl, wie aus dem Diagramm ersichtlich ist, bereits etwas früher abfällt. Bei den mit 4 und 5 bezeichneten Kennlinien wird dagegen, wie unmittelbar ersichtlich, keine Drehzahlregelung vorgenommen.

Je nach Anwendungsfall bzw. erkanntem Belastungszustand des chirurgischen Werkzeugs W kann es sinnvoll sein, das Steuerungs- bzw. Regelungsprofil der Motorsteuerung MS auch auf andere Weise als aus dem Diagramm der Fig. 3 ersichtlich einzustellen. Folgende Steuerungs- bzw. Regelungsprofile seien als Beispiel genannt:

1.) Keine Drehzahlregelung verwenden;
2.) Drehzahlregelung mit veränderten Regelparametern verwenden;
3.) Drehzahlregelung mit optimal abgestimmten Parametern, jedoch den Sollwert der Drehzahlregelung situationsabhängig anpassen;
4.) Änderung des Motorinnenwiderstandes:

Proportional zum gemessenen Motorstrom senkt die Motorsteuerung MS die Ausgangsspannung für den Elektromotor M wie folgt ab.

$$U_{korrigiert} = U_{normal} - \Delta U$$

$$\Delta U = R \times I_{Motor}$$

Bei einem Elektromotor M mit hohem Innenwiderstand fällt die Spannung $\Delta U$ am Innenwiderstand der Wicklung ab. Die Motorsteuerung MS gibt daher eine um $\Delta U$ verringerte Spannung aus, um die gleiche Wirkung an einem Motor mit nicht so hohem Innenwiderstand zu erreichen.

5.) In der Parameter-Vorgabeeinrichtung PV ist ein geeigneter Lernalgorithmus implementiert, der das Regelungsprofil des Elektromotors nach Maßgabe durch Lernen des Belastungszustands des Elektromtors einstellt. Beispielsweise erfasst die Parameter-Vorgabeeinrichtung PV über ein Temperaturmodell die Motorerwärmung. Speziell bei längerem Motorbetrieb, wie er beim Fräsen auftritt, wird darauf geschlossen, dass das Werkzeug W für Fräsarbeiten eingesetzt wird, worauf die Motorsteuerung MS auf das hierfür am besten geeigneten Steuerungs- bzw. Regelungsprofil umgeschaltet wird.

Da die von der Sensorik S erzeugten Signale je nach Art der Motorsteuerung MS auch von dieser als Rückkopplungswerte benötigt werden (wie in Fig. 1 durch die gestrichelte Linie angedeutet ist), genügt eine einzige Sensorik für die Motorsteuerung MS und die erfindungsgemäß Parameter-Vorgabeeinrichtung PV. D.h., da eine Motorsteuerung MS der hier benötigten Art in der Regel bereits eine Sensorik enthält, werden für die erfindungsgemäße Parameter-Vorgabeeinrichtung PV meist keine zusätzlichen Sensoren benötigt.

## Patentansprüche

1. Antriebssteuereinrichtung für ein chirurgisches Motorensystem mit einer Motorsteuerung (MS) zum Steuern und/oder Regeln eines ein chirurgisches Werkzeug (W) antreibenden Elektromotors (M), **gekennzeichnet durch** eine der Motorsteuerung (MS) vorgeschaltete Parameter-Vorgabeeinrichtung (PV), die auf der Grundlage eines erfassten Zustandes oder Zustandsverlaufs des Elektromotors (M) des chirurgischen Motorensystems die momentane medizinische Applikation ermittelt, einen für diese medizinische Applikation geeigneten Steuerungsmodus auswählt und der Motorsteuerung (MS) solche Steuer- bzw. Regelungsparameter vorgibt, dass das Steuerungs- bzw. Regelungsprofil des Elektromotors (M) dem ausgewählten Steuerungsmodus entspricht.

2. Antriebssteuereinrichtung nach Anspruch 1, **dadurch gekennzeichnet, dass** die Parameter-Vorgabeeinrichtung (PV) den Steuerungsmodus aus einer Vielzahl an hinterlegten und jeweiligen medizinischen Applikationen zugeordneten Steuerungsmodi auswählt.

3. Antriebssteuereinrichtung nach einem der vorhergehenden Ansprüche, **dadurch gekennzeichnet, dass** die Parameter-Vorgabeeinrichtung (PV) in Abhängigkeit des verwendeten chirurgischen Werkzeugs (W) einen diesem Werkzeug zugeordneten Steuerungsmodus auswählt.

4. Antriebssteuereinrichtung nach einem der vorhergehenden Ansprüche, **dadurch gekennzeichnet, dass** jedem Steuerungsmodus eine vorbestimmte Drehmoment/Drehzahl-Kennlinie des Elektromotors (M) zugeordnet ist.

5. Antriebssteuereinrichtung nach einem der vorhergehenden Ansprüche, **dadurch gekennzeichnet, dass** die Parameter-Vorgabeeinrichtung (PV) den jeweiligen Zustand des chirurgischen Motorensystems durch, vorzugsweise kontinuierliche, Erfassung des jeweiligen Momentanwerts von Motor-Strom (I) und Motor-Spannung (U) des Elektromotors (M) bestimmt.

6. Antriebssteuereinrichtung nach Anspruch 5, **dadurch gekennzeichnet, dass** die Parameter-Vorgabeeinrichtung (PV) die Temperatur (T) des Elektromotors (M) durch Integration der erfassten Momentanwerte von Motor-Strom (I) und Motor-Spannung (U) des Elektromotors (M) bestimmt.

7. Antriebssteuereinrichtung nach einem der vorhergehenden Ansprüche, **dadurch gekennzeichnet, dass** zumindest ein Steuerungsmodus eine Drehzahlregelung des Elektromotors (M) vorsieht und zumindest ein Steuerungsmodus keine Drehzahlregelung des Elektromotors (M) durchführt.

8. Antriebssteuereinrichtung nach einem der vorhergehenden Ansprüche, ferner **gekennzeichnet durch** eine Rotorpositionsbestimmungseinrichtung zum Bestimmen einer Rotorposition des Elektromotors, wobei zur Bestimmung der Position des Rotors des Elektromotors mindestens eine der Motorwicklungen für ein vorbestimmtes Zeitintervall von einer Energieversorgung abgetrennt wird.

9. Chirurgisches Motorensystem mit
einem Elektromotor (M) zum Antrieb eines chirurgischen Werkzeugs (W), das mit diesem unmittelbar oder mittelbar, insbesondere unter Zwischenschaltung eines Werkzeugadapters oder Handstücks, lösbar koppelbar ist, und
einer Steuereinrichtung nach einem der Ansprüche 1 bis 8.

10. Chirurgisches Motorensystem nach Anspruch 9, ferner **gekennzeichnet durch** eine Drehzahlerfassungseinrichtung zum Erfassen der Ist-Drehzahl des Elektromotors (M), insbesondere Hall-Sensoren, und/oder eine Handstück- oder Werkzeugerkennungseinrichtung vorgesehen ist.

11. Verfahren zum Steuern und/oder Regeln eines Elektromotors (M) eines chirurgisches Motorensystems, der ein chirurgisches Werkzeug (W) antreibt, **gekennzeichnet durch** die Schritte:

> Erfassen eines Zustands und/oder Zustandsverlaufs des Elektromotors (M) des chirurgischen Motorensystems;
> Ermitteln der momentanen medizinischen Applikation des chirurgischen Werkzeugs (W) auf der Grundlage des erfassten Zustands oder Zustandsverlaufs;
> Auswählen eines Steuerungsmodus auf der Grundlage der ermittelten medizinischen Applikation; und
> Einstellen der Steuer- bzw. Regelungsparameter, so dass das Steuerungs- bzw. Regelungsprofil des Elektromotors (M) dem ausgewählten Steuerungsmodus entspricht.

**Claims**

1. A drive control device for a surgical motor system comprising a motor control unit (MS) for controlling an electric motor (M) in an open and/or closed loop, said electric motor driving a surgical tool (W), **characterized by**
a parameter specification device (PV) which is provided upstream of the motor control unit (MS) and determines the current medical application on the basis of a detected state or state history of the electric motor (M) of the surgical motor system, selects a control mode which is suitable for said medical application and gives the motor control unit (MS) such open or closed loop parameters that the open or closed loop control profile of the electric motor (M) corresponds to the selected control mode.

2. The drive control device according to claim 1, **characterized in that** the parameter specification device (PV) selects the control mode from a plurality of control modes which are deposited and assigned to respective medical applications.

3. The drive control device according to any of the preceding claims, **characterized in that** the parameter specification device (PV) selects a control mode, associated with said surgical tool, depending on the employed surgical tool (W).

4. The drive control device according to any of the preceding claims, **characterized in that** a predetermined torque/speed characteristic of the electric motor (M) is assigned to each control mode.

5. The drive control device according to any of the preceding claims, **characterized in that** the parameter specification device (PV) determines the respective state of the surgical motor system by preferably continuously detecting the respective instantaneous values of motor current (I) and motor voltage (U) of the electric motor (M).

6. The drive control device according to claim 5, **characterized in that** the parameter specification device (PV) determines the temperature (T) of the electric motor (M) by integration of the detected instantaneous values of motor current (I) and motor voltage (U) of the electric motor (M).

7. The drive control device according to any of the preceding claims, **characterized in that** at least one control mode provides for a rotational speed regulation of the electric motor (M) and at least one control mode does not perform a rotational speed regulation of the electric motor (M).

8. The drive control device according to any of the preceding claims, further **characterized by** a rotor position determination means for determining a rotor position of the electric motor, wherein, for the determination of the position of the rotor of the electric motor, at least one of the motor windings is cut off from an energy supply for a predetermined time interval.

9. A surgical motor system, comprising
an electric motor (M) for driving a surgical tool (W) which can be detachably coupled to said motor directly or indirectly, in particular by interposing a tool adapter or a handpiece, and
a control device according to any of claims 1 to 8.

10. The surgical motor system according to claim 9, further **characterized by** providing a rotational speed detection means for detecting the actual speed of the electric motor (M), in particular Hall sensors, and/or a handpiece identification or tool identification means.

11. A method of controlling an electric motor (M) of a surgical motor system in an open and/or closed loop, said electric motor driving a surgical tool (W), **characterized by** the steps:

detecting a state and/or a state history of the electric motor (M) of the surgical motor system;
determining the current medical application of the surgical tool (W) on the basis of the detected state or state history;
selecting a control mode on the basis of the determined medical application; and
adjusting the open or closed loop parameters such that the open or closed loop control profile of the electric motor (M) corresponds to the selected control mode.

**Revendications**

1. Dispositif de commande d'entraînement pour un système chirurgical à moteur doté d'une commande de moteur (MS) pour commander et/ou régler un électromoteur (M) entraînant un outil chirurgical (W), **caractérisé par**
un dispositif de prescription de paramètre (PV) en amont de la commande de moteur (MS) qui, sur la base d'un état ou une évolution d'état détecté(e) de l'électromoteur (M) du système chirurgical à moteur, identifie l'application médicale momentanée, choisit un mode de commande approprié pour cette application médicale et prescrit à la commande de moteur (MS) des paramètres de commande ou de réglage tels que le profil de commande ou de réglage de l'électromoteur (M) correspond au mode de commande choisi.

2. Dispositif de commande d'entraînement selon la revendication 1, **caractérisé en ce que** le dispositif de prescription de paramètre (PV) choisit le mode de commande parmi une pluralité de modes de commande affectés à des applications médicales enregistrées et respectives.

3. Dispositif de commande d'entraînement selon l'une des revendications précédentes, **caractérisé en ce que** le dispositif de prescription de paramètre (PV) choisit en fonction de l'outil chirurgical (W) utilisé un mode de commande affecté à cet outil.

4. Dispositif de commande d'entraînement selon l'une des revendications précédentes, **caractérisé en ce que**, à chaque mode de commande, est affecté un moment de rotation/une courbe caractéristique de vitesse de rotation prédéterminé(e) de l'électromoteur (M).

5. Dispositif de commande d'entraînement selon l'une des revendications précédentes, **caractérisé en ce que** le dispositif de prescription de paramètre (PV) détermine l'état respectif du système chirurgical à moteur par la détection, de préférence continue, de la valeur momentanée respective de courant de moteur (I) et de tension de moteur (U) de l'électromoteur (M).

6. Dispositif de commande d'entraînement selon la revendication 5, **caractérisé en ce que** le dispositif de prescription de paramètre (PV) détermine la température (T) de l'électromoteur (M) par intégration des valeurs momentanées détectées de courant de moteur (I) et de tension de moteur (U) de l'électromoteur (M).

7. Dispositif de commande d'entraînement selon l'une des revendications précédentes, **caractérisé en ce qu'**au moins un mode de commande prévoit un réglage de vitesse de rotation de l'électromoteur (M) et au moins un mode de commande ne réalise pas de réglage de vitesse de rotation de l'électromoteur (M).

8. Dispositif de commande d'entraînement selon l'une des revendications précédentes, **caractérisé en outre par** un dispositif de détermination de position de rotor pour déterminer une position de rotor de l'électromoteur, dans lequel, pour la détermination de la position du rotor de l'électromoteur, au moins l'un des bobinages moteur pour un intervalle temporel prédéterminé est séparé d'un approvisionnement énergétique.

9. Système chirurgical à moteur, comprenant
un électromoteur (M) pour entraîner un outil chirurgical (W) qui peut être couplé à celui-ci de façon amovible directement ou indirectement, en particulier avec interposition d'un adaptateur d'outil ou d'une pièce à main, et
un dispositif de commande selon l'une des revendications 1 à 8.

10. Système chirurgical à moteur selon la revendication 9, **caractérisé en outre en ce qu'**un dispositif de détection de vitesse de rotation pour détecter la vitesse de rotation réelle de l'électromoteur (M), en particulier des capteurs à effet Hall, et/ou un dispositif de reconnaissance de pièce à main ou d'outil est prévu.

11. Procédé de commande et/ou de réglage d'un électromoteur (M) d'un système chirurgical à moteur, qui entraîne un outil chirurgical (W),
**caractérisé par** les étapes de :

détection d'un état et/ou une évolution d'état de l'électromoteur (M) du système chirurgical à moteur ;
identification de l'application médicale momentanée de l'outil chirurgical (W) sur la base de l'état ou évolution d'état détecté(e) ;
choix d'un mode de commande sur la base de l'application médicale identifiée ; et
ajustement des paramètres de commande ou de réglage de sorte que le profil de commande ou de réglage de l'électromoteur (M) correspond au mode de commande choisi.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006012991 A1
- DE 102009018143 A1
- EP 2324779 A1